# EUROPEAN PATENT SPECIFICATION

(11) **EP 4 221 806 B1**
(45) Date of publication and mention of the grant of the patent: **06.11.2024**
(21) Application number: 21815643.8
(22) Date of filing: 07.10.2021
(51) Int. Cl.: A61M 37/00, A61K 41/00, A61K 49/22, A61K 9/00

(54) **SYSTEM FOR STIMULATING IMMUNE RESPONSE AGAINST AN EXISTING CANCER IN A PATIENT**
SYSTEM ZUR STIMULATION DER IMMUNANTWORT GEGEN EINEN BESTEHENDEN KREBS BEI EINEM PATIENTEN
SYSTÈME POUR STIMULER UNE RÉPONSE IMMUNITAIRE CONTRE UN CANCER EXISTANT CHEZ UN PATIENT

(30) Priority: 07.10.2020 US 202063088851 P
(43) Date of publication of application: 09.08.2023
(73) Proprietor: Surf Technology AS, 7491 Trondheim (NO)
(72) Inventor: AMINI, Seyednaseh, 7035 Trondheim (NO); ANGELSEN, Bjorn, 7051 Trondheim (NO); LILLEBY, Wolfgang, 0383 Oslo (NO); SOLBERG, Stian, 5305 Florvåg (NO)
(74) Representative: Alpspitz IP
(86) International application number: PCT/IB2021/000692
(87) International publication number: WO 2022/074458

(56) References cited:
- US-A1- 2009 087 384
- US-A1- 2018 289 805
- US-A1- 2020 246 179

## Description

### Field of the Invention

The invention addresses instrumentation for using ultrasound vibrations of intra-capillary micro-bubbles to increase streaming of lymphatic fluid from primary cancer tumors to draining lymph-nodes (DLN) to increase the generation of tumor-specific CD8+ and CD4+ effector cells for the purpose of enhanced primary ablative and abscopal-immune-related therapy of cancer.

### Discussion of Related Art

Currently, 5000 Norwegian men are diagnosed annually with prostate cancer (PCa), and 1000 men die from the disease each year. It is one of the most prevalent cancers and is now passing 50.000 men in Norway Cancer Registry. In the current clinical practice, there is convincing evidence that early chemotherapy can extend life by several months when applied together with castration (androgen deprivation therapy, ADT) in patients with metastatic PCa. In 2015, the CHAARTED study showed that a combination of docetaxel and castration increased the overall survival with 22 months compared to men receiving only castration in men with primary metastatic prostate cancer [1,2]. However, eventually all men will progress and succumb to metastatic castrate resistant prostate cancer. Recently, the STAMPEDE trial showed a clinical benefit when adding local radiation to the primary tumor in low tumor burden patients [3]. In this trial radiation was applied to the prostate gland only, leaving out other sites of metastatic spread.

Traditionally, the prostate gland is regarded as an immunologically privileged organ not accessible for immunotherapy, partly due to the presence of strongly immune-suppressive molecules like TGFb in prostatic cells, which hinder T-cell access and cytotoxic responses at tumor sites. However, several traits make PCa especially attractive for a combination of radiotherapy with immunotherapy, as the prostate
- is a non-essential organ
- has slow proliferating tumor kinetic
- can be monitored with Prostate-specific antigens (PSA) measured in peripheral blood.

One major challenge consists of abandoning the barrier of immunologic tolerance of the prostate and to find solutions to circumvent how cancerous cells evade T-cell attack. Recent studies linking DNA damage with radiation-mediated immunogenicity provided mechanistic evidence that the immune-modulatory properties of radiotherapy decisively contribute to its therapeutic efficacy. Histologic dissection of the tumor microenvironment may pave the way to achieve this goal. Antigen-presenting cells (APCs) are a heterogeneous group of immune cells that mediate the cellular immune response by processing and presenting antigens for recognition by certain lymphocytes such as T cells. While depletion of regulatory T cells and checkpoint blockades are suggested to broadly license tumor APCs [4], there has been no evidence of strongly stimulatory APCs within the native tumor. Classical APCs include dendritic cells, macrophages, monocytes and B cells. Monocytes can internalize antigens, migrate to lymph nodes and differentiate to inflammatory activated monocytes that act as antigen-presenting cells. The mechanism of antigen capture in lymph nodes and delivery to follicular DC has been under investigation for many decades [5]. The tumor-associated draining lymph nodes (DLN) are essential to the generation of tumor-specific CD8+ and CD4+ effector cells. Antigens are processed by classical dendritic cells in the lymph node. Inside the lymph node antigens exposure trigger T cell responses to both CD4+ and CD8+ T cells [6].

Primary immune responses to a few tumor cells can be induced and augmented by increased tumor-associated antigen (TAA) and neo-antigen presented by professional APCs/DCs to the DLNs after radiation of the prostate. Stereotactic radiotherapy enhances cross-presentation of TAA in the DLNs as the major mechanism by which radiotherapy promotes antigen-specific immune responses and synergizes with immunotherapy [7]. Interestingly, exosomes derived from breast cancer cells treated with the topoisomerase I inhibitor topotecan were reported to produce exosomes that carry DNA capable to activate DCs in a STING-dependent way [8], suggesting that action-induced DNA damage may be coupled with the activation of innate immunity via a common pathway.

Radiation depending on the fraction size increases the expression of several antigens and expands the antigenic repertoire of cancer cells [9,10]. DNA exonuclease Trex1 is induced by radiation doses above 12-18 Gy in different cancer cells, and attenuates their immunogenicity by degrading DNA that accumulates in the cytosol upon radiation. However, the optimal degradation of dsDNA and loading of DCs varies for different cancer cells [11]. Solid tumors have high interstitial pressure which will be altered during the therapy by releasing of cell fragments. TAA will attract DC and these loaded antigen-presenting cells will return to the DLNs.

US2020246179 describes a system for cancer treatment using delivered nanoparticles and ultrasound to heat the tumor and nanoparticles thereby enhancing treatment.

The invention presents instrumentation that utilizes one or both of the extra-capillary acoustic radiation force (ARF) and micro-shear waves produced by intra-capillary vibrating micro-bubbles to increase the outflow of the interstitial lymphatic fluid with TAA- loaded DCs to the lymphatic organs constituting a backflow of primed returning cytotoxic T-cells to both the primary and metastatic tumor deposits constituting a local and an abscopal immune response.

### Summary of the Invention

An overview of the invention is presented, where the overview is a short form and by no means represents limitations of the invention, which in its broadest aspect is defined by the claims appended hereto.

The invention presents instrumentation for increasing immune response against an existing cancer in a patient, comprising
a) means for a primary action of the primary tumor of the patient in a way that generates professional APCs/DCs with tumor associated antigens (TAA) of the primary cancer cells in the interstitial fluid of the primary cancer region, and
b) means for with a selectable delay after the start of said primary action to perform a secondary action, said means comprising
   i) means for introducing micro-bubbles into the tumor capillary system of the cancer region, and
   ii) means for vibrating said micro bubbles with incident ultrasound beams with appropriate frequency and amplitude,
for the purpose of increasing flow of lymphatic fluid containing APCs/DCs with TAA from the primary tumor to the draining lymph nodes (DLNs).

The invention further describes more details of the system where
- said means for primary action includes means for chemical- or radio-pharmaceutical therapy, stereotactic radiotherapy, proton-therapy, and brachytherapy. For chemical- and radio-pharmaceutical therapy there is a specialization that said secondary therapy is delayed until the chemical- or radio-pharmaceutical drug concentration in the blood is below a defined limit, and
- said means for primary action includes means for carrying through multiple primary actions with selected intervals in time, and
- said means for secondary action includes means for carrying through said secondary action multiple times following a primary action, and
- said means for introducing micro-bubbles includes means for fluid micro-droplets into the blood, and means for evaporating said micro-droplets by incident ultrasound, and
- said means for introducing micro-bubbles includes means for injection into the blood of one of i) ultrasound contrast agent micro-bubbles and ii) micro-bubbles with diameter above 5 microns, and
- said means for vibrating said micro-bubbles includes means for ultrasound vibration of said micro- bubbles at a frequency lower than 2 MHz, and means for imaging the cancer region and said micro-gas bubbles at a frequency higher than 2 MHz, and
- where said means for imaging comprises
- means for transmitting co-propagating pulse complexes comprising overlapping low frequency (LF) and high frequency (HF) pulses, said LF pulses are used to nonlinearly manipulate the object properties observed by the co-propagating HF pulse, and
- means for utilizing the nonlinear manipulation of the object properties by the LF pulse on the scattered signal by the co-propagating HF pulse, and
- means for utilizing the scattered signal to produce images of the cancer region and micro-bubbles.

Said system further comprising,
- means for determining the required amplitude of the micro-bubble vibrations to produce a maximal amplitude of the capillary wall vibrations to be within a selected interval, and
- where said means for vibrating said micro bubbles includes
- means for measuring the vibration amplitude of said micro-bubbles, and
- means for using and the measured amplitude as input for adjusting the transmit amplitude of said ultrasound beams.

### Brief Description of the Drawings

**FIG. 1** Illustrates a schematic overview of a cancer region with an example capillary connecting the arteriolar inflow and the venule outflow, together with lymph capillary outflow. The capillary contains a micro-bubble that can be brought into vibrations by incident ultrasound to produce extra-capillary acoustic radiation force and micro-shear waves, and
**FIG. 2a** Illustrates in grey-scale the simulated extra-capillary vibration displacement amplitude produced by the intra-capillary micro-bubble, and
**FIG. 2b** Illustrates in grey-scale the simulated resulting extra-capillary acoustic radiation force produced by the extra-capillary vibrations, and
**FIG. 3a** Illustrates simulated streamlines for the interstitial fluid streaming produced by an intra-capillary vibrating 6 µm diameter micro-bubble, and
**FIG. 3b** Illustrates simulated streamlines for the interstitial fluid streaming produced by an intra-capillary vibrating ACT micro-bubble, and
**FIG. 4** The bars illustrate the flow from the proximal capillary into the interstitium equal to the sum of the back-flow from the interstium into the distal capillary and also into the lymphatic capillaries, and
**FIG. 5a** Illustrates the displacement amplitude of extra-capillary micro-shear waves produced by an intra-capillary vibrating 6 *µm* diameter micro-bubble, and
**FIG. 5b** Illustrates the displacement amplitude of extra-capillary micro-shear waves produced by an intra-capillary vibrating PFC-droplet bubble, and
**FIG. 6a** Illustrates an overview block diagram of a system for according to the invention.
**FIG. 6b** Illustrates a detailed block diagram of an ultrasound system for combined imaging of tumor and micro-bubbles, and also vibration of intra-capillary micro-bubbles for therapy.
**FIG. 6c** Illustrates more details of a 3D probe and mounting the probe in a robotic arm for both 3D imaging and 3D steering of therapy beams.

### Detailed Description of Embodiments

This section gives a more detailed description of example embodiments of the invention, where the examples by no means represents limitations of the invention, which in its broadest aspect is defined by the claims appended hereto.

Radiation therapy on primary prostate cancer or in combination with CTLA-4 has in several patients shown to improve immune response towards metastatic cancer cells [13]. An hypothesis is that the radiotherapy of the primary cancer generates professional Antigen-Presenting Cells/Dendritic Cells (APCs/DCs) with tumor-associated antigen (TAA) to the primary tumor that follows lymphatic flow from the primary tumor to the draining lymph nodes (DLNs) in the patient. The radiation therapy also breaks down the tumor structure that reduces the intra-tumor pressure and improves lymphatic flow and also leaks APCs/DCs with TAAs to interstitial fluid (IF) of near surrounding tissue that also increases lymphatic flow with APCs/DCs with TAAs to the DLNs. This introduction of TAA to the DLNs stimulates the production of tumor specific CD8+ and CD4+ T-cells in the DLNs that follows the lymphatic flow to the veins and are brought back via the blood circulation to the primary tumor and also metastatic tumor residuals, and stimulate increased immune response for killing the cancer cells both in the primary and the distant metastatic deposits (abscopal effect) [12].

The invention presents instrumentation for increasing immune response against an existing cancer in a patient by increasing lymphatic flow from the cancer region to the DLNs, using ultrasound vibrations of micro-bubbles in the cancer region. We define a cancer region as comprising the primary tumor with some region around of practical dimension for the action as further discussed below. The invention comprises first to provide a primary action of the primary tumor in a way that generates professional antigen-presenting cells/dendritic cells (APCs/DCs) with tumor-associated antigen (TAA) from the primary tumor in the interstitial fluid (IF) in the primary tumor and also preferentially in the IF of tissue around the primary tumor which has larger lymphatic drainage to the DLNs and helps to increase the lymphatic drainage of APCs/DCs with TAA to the DLNs. The start of the primary action is followed by a secondary action comprising i) introducing micro-bubbles into the capillary system of the cancer region, and ii) vibrating said micro bubbles with incident ultrasound beams with appropriate frequency and amplitude onto said cancer region, for the purpose of increasing lymphatic flow of APCs/DCs with TAAs from the primary tumor out of the cancer region to its DLNs. The cancer region can also comprise several tumors within fairly healthy tissue, like typically found in early stage prostate cancer, or one cancer tumor can fill the whole region, potentially also including near-by normal tissue.

The vibration of the intra-capillary micro-bubbles produces vibrations in the extra-capillary tissue that produces an outward acoustic radiation force (ARF) from the capillaries that drives fluid from the capillaries out through the tissue interstitium, increasing the interstitial pressure that further increases the flow of lymphatic fluid out from the cancer region to the DLNs. The tissue vibrations also generate micro-shear waves in the extra-capillary tissue with the potential to break elements of the extracellular matrix (ECM), and/or reduce the viscosity of the interstitial fluid (IF), for example by breaking down molecular connections within hyaluronan acid (HA), also up to HA gel. These effects further increase the flow of lymphatic fluid containing professional APCs/DCs with TAA out of the cancer region to the DLNs.

Example primary actions that produce such professional APCs/DCs with TAA from the primary tumor are stereotactic radiation-therapy, brachytherapy using implanted radioactive seeds, proton-therapy, chemo-therapy also including radiopharmaceuticals used in the same way as chemical drugs. The availability in the IF of the cancer region of such professional APCs/DCs with TAAs increases with time after the start of the primary action, and the secondary action with introduction and vibration of intra-capillary micro-bubbles can hence in many cases be done with a selectable delay after the primary action, also multiple times after the primary action and in the interval between primary actions. Vibration of intra-capillary micro-bubbles to improve drug delivery with primary chemical- or radio-pharmaceutical therapy, is currently also under investigation by **Exact Therapeutics,** Oslo [14-16], where we note that the secondary action according to the invention is also useful to increase the flow of lymphatic fluid containing such professional APCs/DCs with TAAs to the DLNs, also after a drug concentration in the blood is substantially reduced. The invention also devices to apply the secondary action up to multiple times between primary actions, to increase the transport of APCs/DCs with TAAs to the DLNs to further increase the immune response towards the cancer. We also note that micro-bubble vibrations in the primary tumor also increases the fluid flow from proximal capillaries into the interstitium of the tumor, further increasing the flow-loop of cytotoxic CD8+/CD4+ T-cells from the blood to the tumor cells that further enhances the immune response to the cancer. Added vibrations of micro-bubbles in the metastatic tumors can also be done to improve the transport of cytotoxic CD8+/CD4+ T-cells from the blood to the metastatic tumor cells.

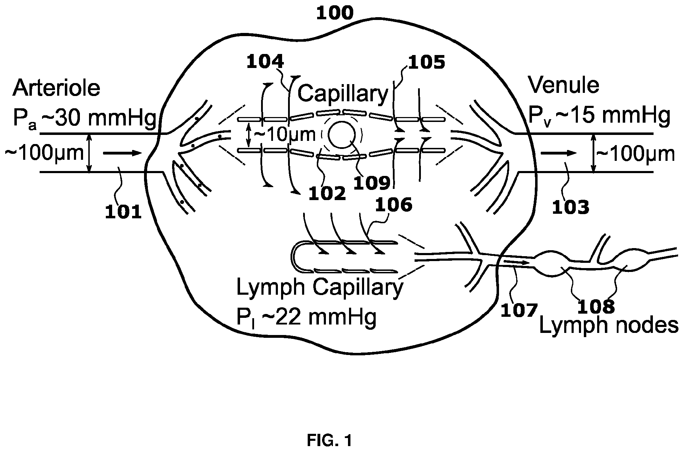

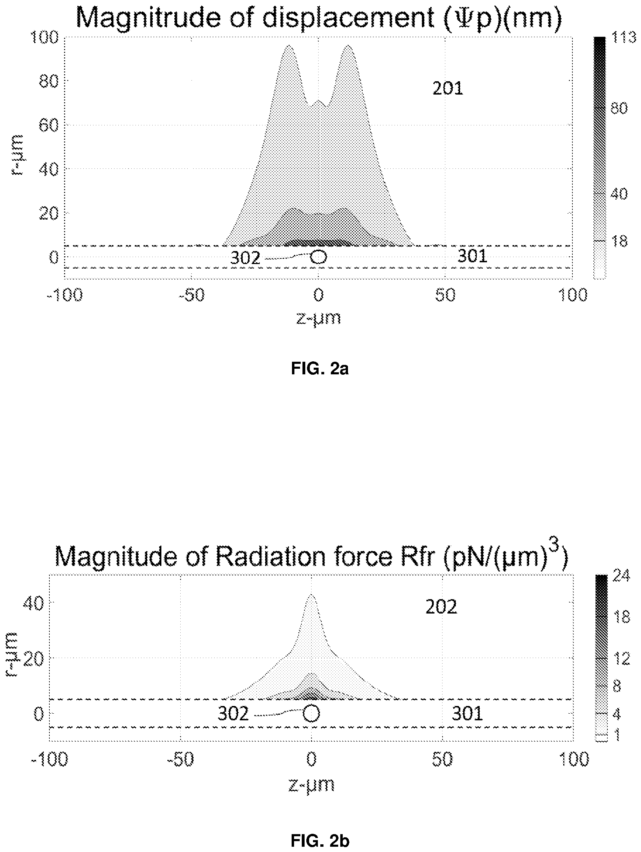

To describe essential features of the invention we start with **FIG.** 1 that is a basis for simulations of some steps of the invention further described in **FIG. 2-5****.** **FIG. 1** shows a schematic drawing of a cancer region **100** in soft tissue. As described above, the cancer region is typically larger than the primary tumor, and can comprise several tumors within fairly healthy tissue, like typically found in early stage prostate cancer. A single tumor can also fill the whole cancer region. After the primary action, professional APCs/DCs with tumor-associated antigen (TAA) of the cancer cells, can also leak into the interstitial fluid (IF) of tissue close to the tumors, and we therefore in the secondary action generally target to treat a cancer region larger than the primary tumor. The cancer region is fed with blood through a set of branching arteries where the 2^{nd} last stage in the artery branching is the arterioles with a diameter ~ 100 *µm,* where **101** shows an example arteriole in the region. The arterioles branches further into the smallest vessel, the capillaries with diameter ~ 10 *µm,* where **102** shows one example capillary with length ~ 500 *µm.* The distal part of the capillaries merges into venules of diameter ~ 100 *µm,* the venous correspondence to the arterioles, and shown as **103** in **FIG. 1****.** The capillaries are hence part of both the arterial and the venous systems. In the Figure is by example shown a single capillary that we use to demonstrate essentials of the invention, where anyone skilled in the art can extend the presentation to a network of branching and converging capillaries between the arterioles and the venules as found in soft tissues.

A typical arteriolar blood pressure is ~ 30 mmHg with a pressure drop of ~ 15 mmHg along the capillaries to the venules. Plasma fluid containing oxygen, nutrition and other materials are transmitted as **104** across the proximal wall of capillaries to the surrounding interstitial space (IS), where the cells are bound together with a collagen matrix, the extracellular matrix (ECM). Interstitial fluid (IF) with metabolic product molecules and other are transmitted as **105** back into the distal part of the capillaries. There is hence a similar pressure drop along the IS as along the capillaries, driving the IF from the proximal region of the capillaries through the IS to the distal region of the capillaries. The Figure does not show these details. At a distance from the capillaries one have close to the average IF pressure with a typical value of *P_{I}* ∼ 22 mmHg.

In the Figure is further shown by example one lymph capillary as **106** of diameter ~ 10 *µm* that drains fluid comprising molecules and white blood cells, called the lymphatic fluid (LF), from the region. This drainage has many functions, amongst other as a regulator of interstitial fluid volume (IFV) and interstitial pressure (IP). Lymph capillaries merges into larger lymph vessels **107,** that further passes the LF through a set of draining lymph nodes **108** (DLN) and further to the blood venous system. Cancer tumors do typically have low lymphatic drainage for the center of the tumor, and leakage of the professional APCs/DCs with tumor-associated antigen (TAA) to the tumor periphery and also to the region outside the tumor, therefore increases lymphatic drainage of the APCs/DCs with TAA to the DLNs.

According to the invention, micro bubbles are introduced into the capillaries, with example implementation as described below. In **FIG.** 1 is shown by example an intra-capillary micro-bubble **109** that for the purpose of the invention is set into volume vibrations by an incoming ultrasound beam (not shown). Both pulsed and continuous vibrations can be used for variable effects and purposes. As micro-bubbles one can use contrast agent micro bubbles that are intravenously injected according to known methods. The contrast agent micro-bubbles typically have a distribution of diameters centered around ~ 3 *µm.* For the contrast agent bubbles one can conveniently dimension filter standard bubble samples to extract bubbles with larger diameters, for example diameters ~ 5 *µm.* These bubbles still flow through the capillaries, but with larger vibration effect on the surrounding tissue than the lower diameter micro-bubbles, an effect that is a central part of the invention. A preferred method is to inject per-fluor-carbon (PFC) oil droplets where evaporation of the PFC droplets in the cancer region into micro-bubbles are stimulated by ultrasound radiation of the droplets [14-16]. A droplet diameter ~ 3 *µm* flows freely through the blood capillaries, and expands to a gas bubble of diameter ~ 17 *µm* in a large artery. In a capillary of ~ 10 *µm* diameter it initially fills the capillary for a length ~ 25 *µm* with semi-spherical endings, being slowly absorbed in the blood during a few minutes. In a capillary of ~ 6 *µm* diameter it initially fills the capillary for a length ~ 90 *µm* before being absorbed in the blood.

The vibrations of the intra-capillary micro-bubbles set the extra-capillary tissue into vibrations, where **FIG. 2a** shows example simulation of the amplitude of extra-capillary tissue vibrations in grey scale as **201,** produced by a 6 *µm* diameter micro-bubble **302** in the center of a straight cylindrical capillary **301** of 10 *µm* diameter and 200 *µm* length, used in the simulations. The capillary axis is along the z-axis of the Figure, and the r-axis is the distance from the capillary axis. The tissue oscillation displacement amplitude has in this example 113 *nm* maximal value at the wall. Such tissue vibrations generates an Acoustic Radiation Force (ARF) in the tissue, outwards from the capillary wall, shown as the grey scale image **202** in **FIG. 2b****.** The maximal value of the ARF is in this example 24 *pN*/(*µm*)³ at the wall.

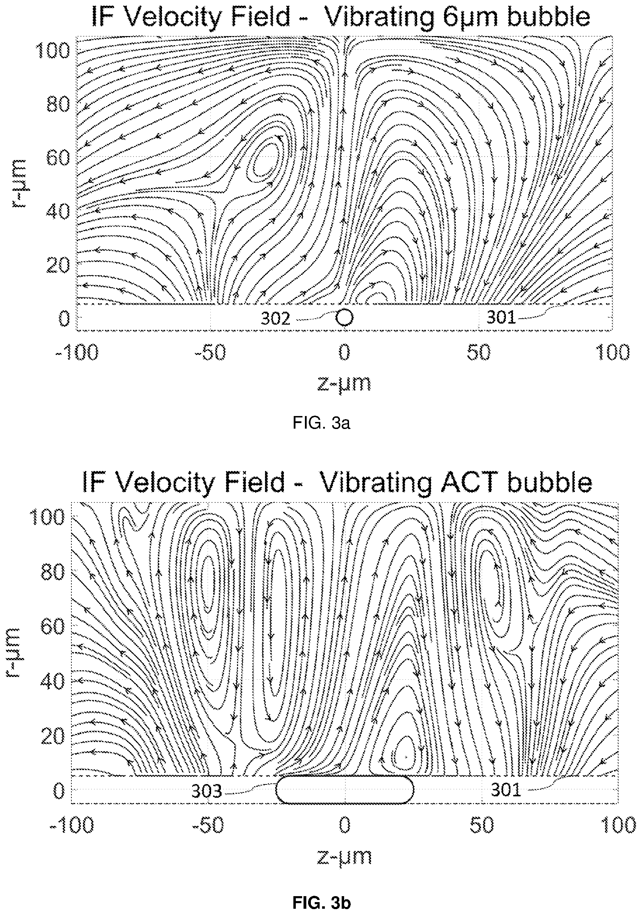

The ARF further interacts with the interstitial fluid (IF) in the tissue. As the IF is incompressible, the localized ARF around the capillary wall produces an IF streaming pattern throughout a large region of the IS, as shown by the simulations for the 6 *µm* diameter bubble in **FIG. 3a** and for an evaporated PFC-droplet bubble filling the capillary for 40 *µm* with semi-spherical endings in **FIG. 3b****. 301** shows the 200 *µm* long capillary with 10 *µm* diameter, where **302** indicates the 6 *µm* contrast agent micro-bubble, and **303** shows the evaporated PFC-droplet bubble, used in the simulations. We note that as the PFC-droplet bubble is directly pressuring against the capillary wall along a large distance, it produces a large wall and tissue displacement that produces a larger ARF IF streaming in the tissue, compared to the bubble **302** not touching the wall. The pressure drop along the capillary is in both cases selected to 15 *mmHg.* The lymphatic capillaries drain the interstitial fluid (IF) to a distal lymphatic region (DLF), with a selected pressure 15 *mmHg* in the simulations. The hydraulic conductivity of the capillary wall is set to 1 *µm*/*s*(*mmHg*) for both out and inflow to the capillary, while the hydraulic conductivity of the lymphatic wall is set to 0.76 *µm*/*s*(*mmHg*). The hydraulic conductivity of the interstitial fluid (IF) through the interstitial space (IS), also referred to as the extracellular matrix (ECM), is set to 1 *µm²*/*s*(*mmHg*) *mm* We note that both Figures show flow from the proximal capillary into the IS, and from the IS back into the distal capillary. Lymphatic flow occurs when the pressure in the IS exceeds the pressure in the DLF. The larger IF streaming with the PFC-droplet bubbles also produces a larger Lymphatic flow with the PFC-droplet bubbles.

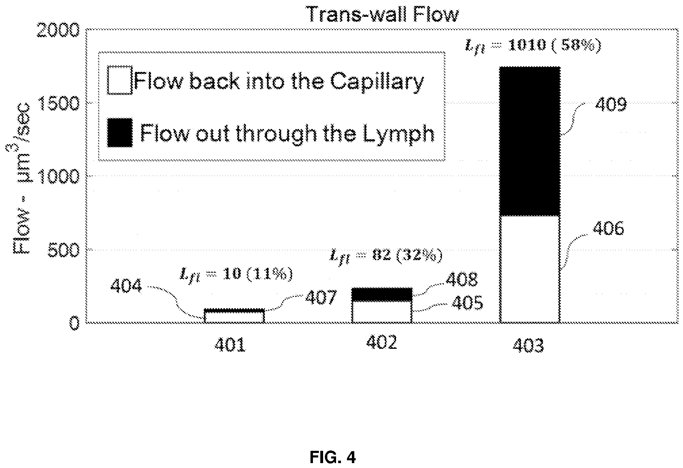

**FIG. 4** shows as the full bars the simulated integrated flow (*µm³*/*s*) out of the proximal part of the capillary for the 200 *µm* capillary without bubble as **401** (91 *µm*³/*sec*), the capillary with a vibrating 6 *µm* bubble in the center as **402** (256 *µm*³/*sec*), and the capillary with a vibrating PFC-droplet bubble in the center as **403** (1740 *µm*³/*sec*). In the stationary situation, this outflow from the proximal capillary to the IS (full bars) matches the sum of the inflow back into the distal capillary, shown as **404** (82 *µm³*/*sec*), **405** (174 *µm³*/*sec*), **406** (731 *µm³*/*sec*), and the outflow through the lymphatic system shown as **407(10** *µm³*/*sec*)*,* **408** (82 *µm*³/*sec*), **409** (1010 *µm³*/*sec*) for the capillary without bubble, capillary with 6 *µm* bubble, and capillary with PFC-droplet bubble. We note that the lymphatic outflow is 11% of total outflow for the open capillary **401,** 32% of for the capillary with 6 *µm* micro-bubble **402,** and 58% of for the capillary with PFC-droplet bubble **403.** The reason for this relative increase in the lymphatic outflow, is the increase in distal interstitial pressure due to the increase in ARF from no micro-bubble **401,** to the 6 *µm* micro-bubble **402,** and to the PFC-droplet bubble **403.** We notice that both the relative amount and the absolute amount of lymphatic flow increases with the introduction of vibrating micro-bubbles, and also the size of the micro-bubbles. The reason for this is that the ARF produced by the vibrating micro-bubbles increases the interstitial fluid pressure (IFP) at a distance from the capillary, increasing the lymphatic outflow from the cancer region. The IFP and the lymphatic outflow increases with the introduction and size of the micro-bubbles. The magnitude of the ARF is proportional to the square of the tissue vibration amplitude, and the ARF hence increases with the size and vibration amplitude of the micro-bubbles.

The rapid growth of the cancer tumor leaves low density of lymph capillaries in the central region of the tumor that produces a weak lymphatic drainage from the central tumor, increasing the interstitial fluid pressure in these regions. In addition the growth produces a high concentration of collagen and elastin in the extra cellular matrix (ECM) of the tumor, that also increase the intra-tumor pressure due to osmotic and capillary forces on the interstitial fluid (IF) between the collagen and elastin molecules. This intra-tumor pressure compresses both lymph and blood capillaries, also increasing both the lymphatic resistance and limiting fluid streaming from the blood capillaries. Radiation therapies and some selected chemo- or radio-pharmaceutical therapy often break down the ECM and tumor cells in the early phase of the therapy, increasing the hydraulic conductivity of the IF, and also presenting professional APCs/DCs with tumor-associated antigen (TAA) of the cancer cells into the near surrounding tissues of the tumor where the lymphatic drainage is better than in the central tumor.

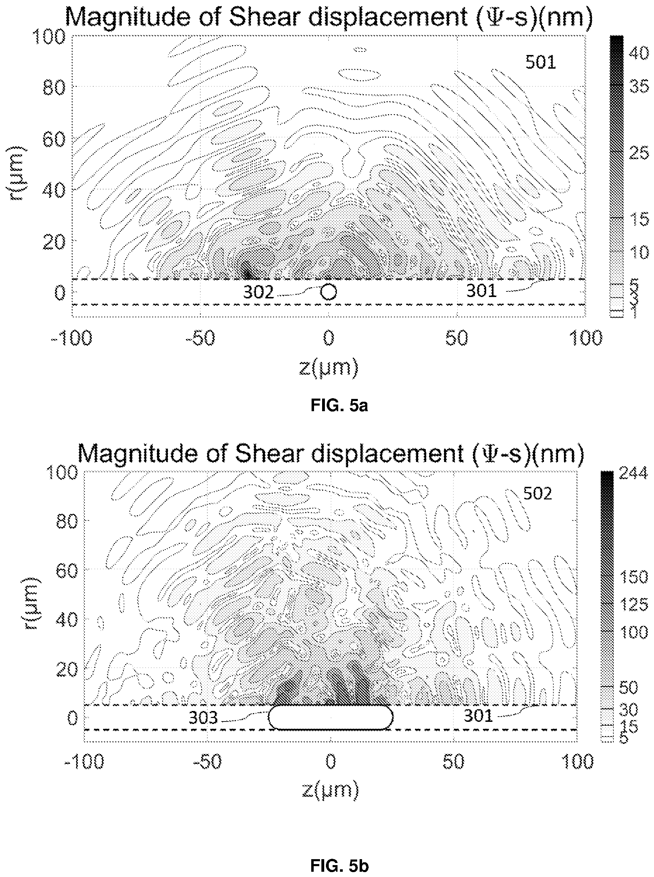

Interaction between heterogeneity in tissue mass and shear stiffness on the cellular scale, and the tissue pressure waves produced by low frequency (~ 500 kHz) ultrasound vibrations of the intra-capillary micro-bubbles, produces additional micro-shear waves in the extra-capillary tissue with a potential to also break down the ECM structure. **FIG. 5** shows example simulations of such extra-capillary micro shear waves in an example tissue, where in **FIG. 5a****, 501** shows in grey-scale the magnitude of the shear wave displacement produced by an intra-capillary vibrating 6 *µm* micro-bubble with maximal displacement of ~ 45 *nm* and maximal shear stress of ~ 2 *kPa* at the capillary wall. **FIG. 5b** shows in grey-scale as **502** the magnitude of the shear wave displacement produced by an intra-capillary vibrating PFC-droplet bubble with maximal displacement of ~ 200 *nm* and maximal shear stress of ~ 30 *kPa* at the capillary wall. The wavelength of these micro-shear waves is ~ 20 *µm @* ~ 500 *kHz.* The amplitude of the shear wave displacement hence increases with the dimension and vibration amplitude of the micro-bubble. Such micro-shear waves have a potential to break parts of the ECM and hence also increase the hydraulic conductivity through the interstitial space and the lymphatic flow, also after the bubble vibrations.

In addition, hyaluronan acid (HA) concentration often increases in diseased tissues like cancer tumors, a phenomenon that also increases the viscosity of the IF in the tumors. The concentration of HA in some cancer tumors can even be so high that the IF forms a gel. The micro shear waves described above have a potential to break down HA molecular cross-coupling, potentially to the level of breaking down HA-gel, reducing the IF viscosity. Such effects does also increase the hydraulic conductivity of the IF, increasing lymphatic outflow from the tumor, both during the limited therapy sessions with ARF from the bubble vibrations, and also for longer periods after the therapy sessions without ARF. We note that this reduction in IF viscosity can last for larger parts after the therapy vibration sessions, potentially up to 24 hr per day, 7 days a week, and hence have a larger effect on increasing the lymphatic flow from the cancer region to the DLNs, compared to the ARF that is found only during the therapy vibration sessions that are typically less than ~ 1 hr with up to days apart.

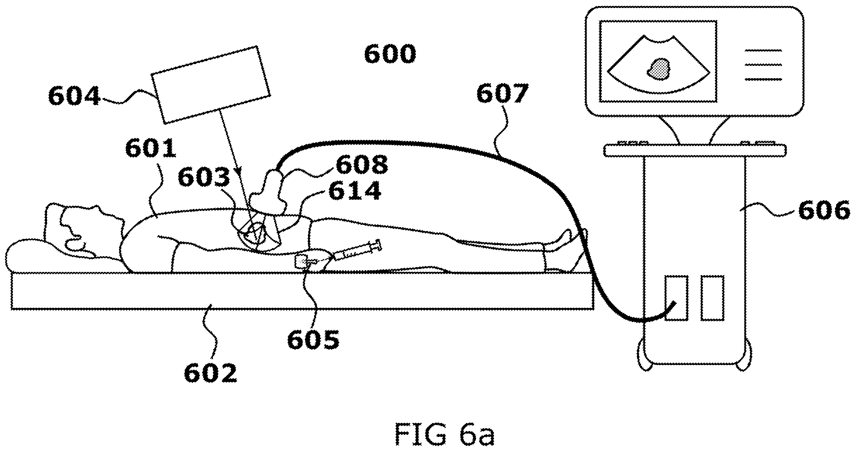

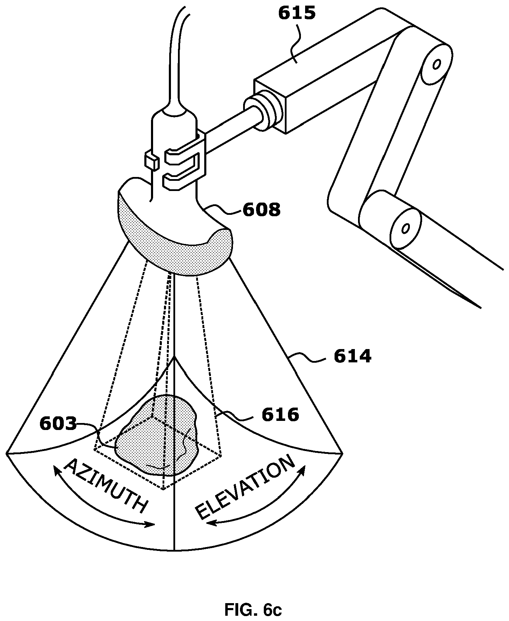

**FIG. 6a** shows components of a system **600** according to the invention. The patient **601** to be treated is placed on a comfortable bed or reclining chair **602,** where **603** indicates the primary tumor. The arrow **604** indicates the principal action applied to the tumor, for example chemical- or radio-pharmaceutical therapy, stereotactic radiation-therapy, brachytherapy produced by implanted radioactive seeds, and proton-therapy. This action produces tumor associated antigens (TAA) of the cancer cells that are found in the interstitial fluid (IF) of the cancer region. The patient **601** is connected to a system **605** for injecting micro-bubbles or primary PFC-droplets as described above. Intra-venous injection is typical, while direct intra-arterial injection is also useful. **606** shows an ultrasound system that connects via a cable **607** to an ultrasound probe **608** that includes arrays of transducer elements capable of transmitting ultrasound pulses for vibration of intra-capillary micro-bubbles as described in relation to **FIG. 1-5****,** and also for imaging of the tumor and the micro-bubbles. The frequency for micro-bubble vibration is typically in the low frequency (LF) range ∼ 0.1 - 2 MHz, while the frequency for imaging of the tumor and micro-bubbles is typically in the high frequency (HF) range ∼ 2 - 50 MHz, depending on the depth of the tumor. The HF imaging frequency is also useful for evaporation of primary PFC-droplets, as described above. It is therefore advantageous to use ultrasound probes with dual arrays for the two frequencies, for example as described in US Patents 7,727,156; 8,182,428; 10,310,061 and US patent publication 2018/0145654**.** The probe should be able to scan the direction of the ultrasound beams, preferably within a 3D space as indicated by the sketch **614** in both **FIG. 6a** and **6c****.** 2D beam scanning can also be useful, especially if the probe is handheld where hand movements of the probe allows adequate pointing of the beams towards the actual parts of the cancer region.

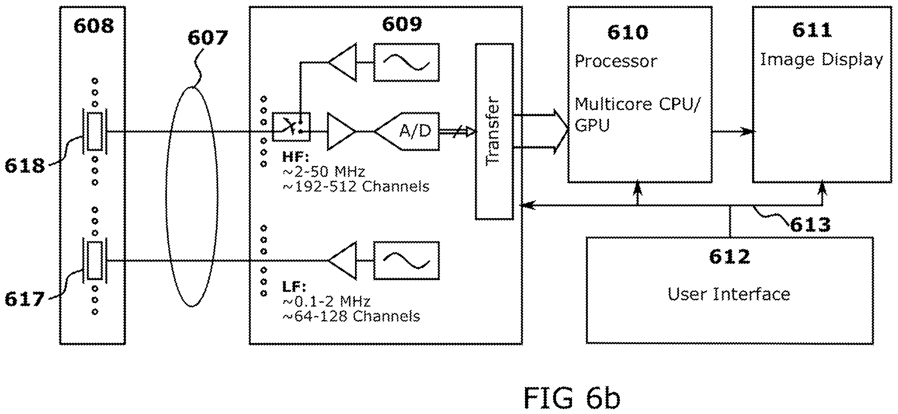

**FIG. 6b** shows how the probe cable **607** connects the probe **608** with LF and HF array ultrasound transducer elements/channels **617** and **618** to a transmit/receive block **609** with multiple transmit/receive channels that for both action and imaging transmits channels of electric transmit pulses for the different LF and HF elements in the transducer arrays of the probe, and for imaging digitizes the HF received signals for all receive channels for further processing to ultrasound images in the processor **610,** for example according to standard methods, or according to the new, dual frequency methods as described in US Patents 7,641,613; 8,038,616; 8,550,998; 8,793,079; 9,291,493; 9,939,413; 10,578,725 and US patent publications 2017/0343656; 2019/0235076; and 2020/0191690. In these new dual frequency imaging methods, one does for imaging transmit dual frequency pulse complexes comprising overlapping LF and HF pulses where the LF pulse is used to nonlinearly manipulate the tissue elasticity observed by the co-propagating HF pulse. This manipulation is accounted for in the processing to provide images with less noise, improved spatial resolution, and also quantitative elasticity data that enhances detection and characterization of the cancer region, as described in the cited patents and patent applications. With today's computer technology the processor can be based on software programmable multicore CPUs (Central Processing Units) and/or GPUs (Graphics Processing Units) according to known methods. The processor provides data for an image display **611,** and the user sets up the system through a user interface **612** that communicates with the other blocks via the communication path **613.**

In a preferred embodiment, the processor analyses the image data to determine regions for therapy beams in front of the probe. A probe **608** with 3D scanning of the beams as illustrated in **FIG. 6c** then provides many practical opportunities. **614** of **FIG. 6c** indicates a typical region of 3D scanning, and the system can from the 3D ultrasound image **603** of the tumor region define a selected region **616** to scan therapy beams. Mounting the probe to a fixture **615** can then allow the therapy beams to follow limited movements of the patient during a therapy session. A robotic arm **615** as fixture for the probe can be used to allow the therapy beam scanning to follow larger movements of the patients.

The invention is effective when the primary action produces professional APCs/DCs with tumor associated antigens (TAA) of the primary cancer cells, that following the primary action are found in the interstitial fluid (IF) of the cancer region in the patient. In the secondary action, micro-bubbles are introduced to the capillary system of the primary tumor and brought to vibrate by incident ultrasound of adequate frequency and amplitude. The micro-bubbles can be of different types, for example, marketed ultrasound contrast agent micro-bubbles, marketed contrast agent micro-bubbles selectively filtered to be above a defined diameter, and PFC-droplet bubbles as presented in [14-16].

After imaging and detecting the primary cancer region, the probe is used to generate therapy beams that vibrate the intra-capillary micro-bubbles. The micro-bubbles are typically vibrated close to or below their resonance frequency to get adequate vibration amplitude for the therapy. For smaller micro-bubbles the therapy beams can be generated by the HF array, while for the larger micro-bubbles where the resonance frequency is in the ~ 500 kHz range, the therapy beams are typically generated with the LF array. The intra-capillary micro-bubble vibrations set up vibrations in the extra capillary tissue that produces an outward acoustic radiation force (ARF) that increases the interstitial pressure and the lymphatic outflow to the primary draining lymph nodes (DLN) of the cancer region. The tissue vibrations also produce micro-shear waves in the extra capillary tissue that can increase the hydraulic conductivity of the IF, and hence also the lymphatic outflow. Professional APCs/DCs with TAA of the cancer cells, produced by the first action with radiation- and/or chemical- or radio-pharmaceutical therapy on the primary tumor, is then found in the interstitial fluid and transported from the primary cancer region to the DLNs by the increased lymphatic flow produced by the secondary action. TAA incorporated in dendritic cells then present antigens at the lymph nodes (DLNs) for priming of tumor reactive CD8+/CD4+ T- cells leading to the expansion of these cell populations. This is essential, and as such has been successfully targeted therapeutically using GM-CSF (Dranoff, 2002) to increase presentation in the DLNs. As such, much of the focus has remained on the DLN despite our clear understanding that antigen-loading occurs within the vicinity of the tumor itself and likely influences the functions of tumor CTLs.

This increased introduction of TAA to the primary DLNs stimulates the production of CD8+ and CD4+ T-cells in the DLNs that are further transported by lymphatic flow to the venous system and brought back via the blood circulation to the primary tumor and also metastatic tumor residuals, to stimulate increased immune response for killing the cancer cells both in the primary and the distant metastatic deposits (abscopal effect). For the tumor where vibrations of the micro-bubbles are in process, the vibrating micro-bubbles also increases the fluid flow from proximal capillaries into the interstitium as in **FIG. 4****,** further increasing the flow of cytotoxic CD8+/CD4+ T-cells from the blood to that tumor that further enhances the immune response in that tumor.

Recent studies linking DNA damage with radiation mediated immunogenicity provided mechanistic evidence that the immune modulatory properties of radiotherapy also contribute to its therapeutic efficacy [2,3]. Within the irradiated tumor micro-environment (TME), radiotherapy initiates a cascade of molecular and cellular events leading to immunogenic cell death, up-regulation of MHC class I, enhanced type I IFN signaling and dendritic cell activation [3,6]. Ultimately, effective anti-tumor adaptive immunity requires the priming and subsequent chemokine- driven trafficking of tumor-specific CD8+CTLs into the TME to mediate tumoricidal effector functions [7-9].

To achieve desired acoustic radiation force and micro-shear wave amplitudes, one wants the tissue vibration amplitude to be within a desired range. For a given type and dimension of the micro-bubbles there are mathematical relations between the micro-bubble vibration amplitude and the vibration amplitudes of the capillary wall and hence also the extra-capillary tissue vibration amplitudes. By example, the PFC-droplet bubble fills the capillary, so that the vibration amplitude of the capillary wall is equal to the vibration amplitude of the PFC-droplet bubble . For smaller micro-bubbles that do not fill the capillary, there are mathematical relations between micro-bubble and the capillary wall vibration amplitudes, determined by the diameters of the micro-bubble and the capillary, the micro-bubble location in the capillary, and also by the shear stiffness of the extra-capillary tissue.

A first stage in the process of defining the required ultrasound transmit amplitude to obtain adequate vibration amplitude of the extra capillary tissue, is hence to determine the required micro-bubble vibration amplitude to obtain adequate vibration amplitude of the tissue. For the PFC-droplet bubbles the capillary wall vibration and bubble vibration amplitudes are the same. For micro-bubbles with diameter less than the capillary diameter, one must first determine the shear stiffness of the tissue, which can be obtained from the well-known shear wave imaging or from the nonlinear bulk elasticity parameter, for example as described in US Patent 9,291,493, US Patent publications 2019/0235076; 2020/0191690; that is correlated to density of collagen, elastin, and other large molecules that also determine the shear stiffness. From a mathematical model one can then calculate the required micro-bubble vibration amplitude, and measure the ultrasound transmit amplitude that produces adequate micro-bubble vibration amplitudes. Imaging the micro-bubbles with the dual frequency ultrasound as described in the cited US Patents and Patent applications, one can use the high frequency (HF) (> 2 MHz) image to determine the micro-bubble vibration amplitude produced by the low frequency (LF) (~ 0.1 - 2 MHz) manipulation pulses. This allows adjustments of the LF transmit amplitude to obtain micro-bubble vibration amplitudes within a desired interval.

Thus, while there have shown and described and pointed out fundamental novel features of the invention as applied to preferred embodiments thereof, it will be understood that various omissions and substitutions and changes in the form and details of the devices illustrated, and in their operation, may be made by those skilled in the art.

It is also expressly intended that all combinations of those elements and/or method steps (not claimed) which perform substantially the same function in substantially the same way to achieve the same results, are within the scope of the invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

### References:

1. Kyriakopoulos CE, Chen YH, Carducci MA, Liu G, Jarrard DF, Hahn NM, et al. Chemohormonal Therapy in Metastatic Hormone-Sensitive Prostate Cancer: Long-Term Survival Analysis of the Randomized Phase III E3805 CHAARTED Trial. J Clin Oncol. 2018;36(11):1080-7.
2. Sweeney CJ, Chen YH, Carducci M, Liu G, Jarrard DF, Eisenberger M, et al. Chemohormonal Therapy in Metastatic Hormone-Sensitive Prostate Cancer. N Engl J Med. 2015;373(8):737-46.
3. Parker CC, James ND, Brawley CD, Clarke NW, Hoyle AP, Ali A, et al. Radiotherapy to the primary tumour for newly diagnosed, metastatic prostate cancer (STAMPEDE): a randomised controlled phase 3 trial. Lancet. 2018;392(10162):2353-66.
4. Curran MA, Montalvo W, Yagita H, Allison JP. PD-1 and CTLA-4 combination blockade expands infiltrating T cells and reduces regulatory T and myeloid cells within B16 melanoma tumors. Proc Natl Acad Sci USA. 2010;107(9):4275-80.
5. Mitchell J, Abbot A. Ultrastructure of the antigen-retaining reticulum of lymph node follicles as shown by high-resolution autoradiography. Nature. 1965;208(5009):500-2.
6. Kvalheim G. Biochemical relapse in very high-risk prostate cancer after radical prostatectomy and DC-vaccine loaded with tumor RNA, hTERT, and survivin. J Clin Oncol. 2020;38(6 Supplement):no pagination.
7. Marciscano AE, Ghasemzadeh A, Nirschl TR, Theodros D, Kochel CM, Francica BJ, et al. Elective Nodal Irradiation Attenuates the Combinatorial Efficacy of Stereotactic Radiation Therapy and Immunotherapy. Clin Cancer Res. 2018;24(20):5058-71.
8. Kitai Y, Kawasaki T, Sueyoshi T, Kobiyama K, Ishii KJ, Zou J, et al. DNA-Containing Exosomes Derived from Cancer Cells Treated with Topotecan Activate a STING-Dependent Pathway and Reinforce Antitumor Immunity. J Immunol. 2017;198(4):1649-59.
9. Reits EA, Hodge JW, Herberts CA, Groothuis TA, Chakraborty M, Wansley EK, et al. Radiation modulates the peptide repertoire, enhances MHC class I expression, and induces successful antitumor immunotherapy. J Exp Med. 2006;203(5):1259-71.
10. Garnett CT, Palena C, Chakraborty M, Tsang KY, Schlom J, Hodge JW. Sublethal irradiation of human tumor cells modulates phenotype resulting in enhanced killing by cytotoxic T lymphocytes. Cancer Res. 2004;64(21):7985-94.
11. Vanpouille-Box C, Demaria S, Formenti SC, Galluzzi L. Cytosolic DNA Sensing in Organismal Tumor Control. Cancer Cell. 2018;34(3):361-78.
12. Nesslinger NJ, Sahota RA, Stone B, et al. Standard treatments induce antigen-specific immune responses in prostate cancer. Clin Cancer Res 2007;13: 1493-502.
13. Parker C, Lancet Oncology, Fizazi K et al. Eur Urol 2020
14. Sontum, PC; Kvåle, S; Healey, A J; Skurtveit, R; Watanabe, R; Matsumura, M:, Østensen, J: Intern Journal of Pharmaceuticcs, 495, 2015, 1019-1027
15. van Wamel, A; Sontum, P C.; Healey, A; Kvåle, S; Bush, N; Bamber, J; Davies, C de L. Journal of Controlled Release, 236, 2016 naive prostate cancer
16. Doinikov, A A; Sheeran P S; Bouakaz A; Dayton P A: Med Phys 41 (10), Oct 2014, 102901-1-10

## Claims

1. A system (600) for increasing immune response against an existing cancer in a patient comprising
a) means for a primary action on the primary tumor of the patient that generates professional antigen-presenting cells APCs/DCs with tumor associated antigens (TAA) of the primary cancer cells in the interstitial fluid of the primary cancer region, and
b) means that for a selectable delay after the start of said primary action performs one or more secondary actions, said means comprising
i) means for introducing micro-bubbles (109) into the tumor capillary system of the cancer region, and
ii) means (608) for vibrating said micro-bubbles with incident ultrasound beams with appropriate frequency and amplitude, for the purpose of increasing flow of lymphatic fluid containing APCs/DCs with TAA from the primary tumor to the draining lymph nodes (DLNs).

2. The system according to Claim **1,** where said means for primary action includes means for chemical- or radio-pharmaceutical therapy.

3. The system according to Claim **2,** where said means for secondary action includes means for delaying said secondary action until the chemical- or radio-pharmaceutical drug concentration in the blood is below a defined limit.

4. The system according to Claim **1,** where said means for primary action includes means for hypo-fractionated stereotactic radiotherapy or proton-therapy.

5. The system according to Claim **1,** where said means for primary action includes means for brachytherapy radiation action.

6. The system according to Claim **1,** where said means for primary action includes means for carrying through multiple primary actions with selected intervals in time,

7. The system according to Claim **1** or **6,** where said means for secondary action includes means for carrying through said secondary action multiple times following a primary action.

8. The system according to Claim **1,** where said means for introducing micro-bubbles includes means for injecting fluid micro-droplets into the blood, and means for evaporating said micro-droplets by incident ultrasound.

9. The system according to Claim **1,** where said means for introducing micro-bubbles includes means for injection into the blood of one of i) ultrasound contrast agent micro-bubbles and ii) micro-bubbles with diameter above 5 microns.

10. The system according to Claim **1,** where said means for vibrating said micro-bubbles includes
- means for ultrasound vibration of said micro-bubbles at a frequency lower than 2 MHz, and
- means for imaging the cancer region and said micro-bubbles at a frequency higher than 2 MHz.

11. The system according to Claim 10, where said means for imaging comprises
- means for transmitting pulse complexes comprising co-propagating and overlapping, low frequency (LF) and high frequency (HF) pulses, said LF pulses are used to nonlinearly manipulate the object properties observed by the co-propagating HF pulse, and
- means for utilizing the nonlinear manipulation of the object properties by the LF pulse on the scattered signal by the co-propagating HF pulse to produce images of the cancer region and micro-bubbles.

12. The system according to Claim **1,** where said means for vibrating said micro-bubbles includes
- means for determining the vibration amplitude of said micro-bubbles with ultrasound, and
- means for using the measured amplitude as input for adjusting the transmit amplitude of said ultrasound beams to obtain a bubble vibration amplitude close to a specified value.

13. The system according to Claim 1, comprising
- means for determining a relation between the micro-bubble vibration amplitude and the corresponding maximal amplitude of the capillary wall vibrations.

## Patentansprüche

1. System (600) zur Erhöhung der Immunreaktion gegen vorhandenen Krebs in einem Patienten, mit
a) einem Mittel für eine Primärmaßnahme gegen den Primärtumor des Patienten, das professionelle Antigen-präsentierende Zellen APCs/DCs mit Tumor zugehörigen Antigenen (TAA) der Primärkrebszellen in der interstitiellen Flüssigkeit der Primärkrebsregion erzeugt, und
b) einem Mittel, das mit einer auswählbaren Verzögerung nach dem Beginn der Primärmaßnahme eine oder mehrere Sekundärmaßnahmen durchführt, wobei das Mittel aufweist
i) ein Mittel zum Einbringen von Mikrobläschen (109) in das Tumorkapillarsystem der Krebsregion, und
ii) ein Mittel (608) zum Vibrieren der Mikrobläschen mittels auftreffender Ultraschallstrahlen mit geeigneter Frequenz und Amplitude, um den Fluss von Lymphflüssigkeit, die APCs/DCs mit TAA enthält, von dem Primärtumor zu den ableitenden Lymphknoten (DLNs) zu erhöhen.

2. System nach Anspruch 1, bei dem das Mittel für die Primärmaßnahme ein Mittel aufweist für eine Chemo- oder Radiopharmazietherapie.

3. System nach Anspruch 2, bei dem das Mittel für die Sekundärmaßnahme ein Mittel aufweist zum Verzögern der Sekundärmaßnahme, bis die Chemomedikamentenkonzentration oder die radiopharmazeutische Medikamentenkonzentration in dem Blut unterhalb eines definierten Grenzwerts ist.

4. System nach Anspruch 1, bei dem das Mittel für die Primärmaßnahme ein Mittel aufweist für eine hypofraktionierte stereotaktische Strahlentherapie oder Protonentherapie.

5. System nach Anspruch 1, bei dem das Mittel für die Primärmaßnahme ein Mittel aufweist für eine Brachytherapiestrahlungsmaßnahme.

6. System nach Anspruch 1, bei dem das Mittel für die Primärmaßnahme ein Mittel aufweist zum Durchführen von mehreren Primärmaßnahmen in ausgewählten Zeitintervallen.

7. System nach Anspruch 1 oder 6, bei dem das Mittel für die Sekundärmaßnahme ein Mittel aufweist zum mehrmaligen Durchführen der Sekundärmaßnahme nach einer Primärmaßnahme.

8. System nach Anspruch 1, bei dem das Mittel zum Einbringen der Mikrobläschen ein Mittel aufweist zum Injizieren von Flüssigkeitsmikrotröpfchen in Blut, und ein Mittel zum Verflüchtigen der Mikrotröpfchen durch einfallenden Ultraschall.

9. System nach Anspruch 1, bei dem das Mittel zum Einbringen der Mikrobläschen ein Mittel aufweist zum Injizieren von i) Ultraschallkontrastmittelmikrobläschen oder ii) Mikrobläschen mit einem Durchmesser größer als 5 Mikrometer in das Blut.

10. System nach Anspruch 1, bei dem das Mittel zum Vibrieren der Mikrobläschen aufweist
- ein Mittel zur Ultraschallvibration der Mikrobläschen bei einer Frequenz unter 2 MHz, und
- ein Mittel zur Bildgebung der Krebsregion und der Mikrobläschen bei einer Frequenz größer als 2 MHz.

11. System nach Anspruch 10, bei dem das Mittel zur Bildgebung aufweist
- ein Mittel zum Senden von Pulskomplexen mit sich co-ausbreitenden und überlappenden Niederfrequenz (LF) und Hochfrequenz (HF) Pulsen, wobei die LF-Pulse verwendet werden zur nichtlinearen Manipulation der Objekteigenschaften, die durch den co-ausbreitenden HF-Puls beobachtet werden, und
- ein Mittel zur Verwendung der nichtlinearen Manipulation der Objekteigenschaften durch den LF-Puls für das gestreute Signal durch den sich co-ausbreitenden HF-Puls, um Bilder der Krebsregion und der Mikrobläschen zu erzeugen.

12. System nach Anspruch 1, bei dem das Mittel zum Vibrieren der Mikrobläschen aufweist
- ein Mittel zum Bestimmen der Vibrationsamplitude der Mikrobläschen mit Ultraschall, und
- ein Mittel zum Verwenden der gemessenen Amplitude als Eingabe zum Einstellen der Sendeamplitude der Ultraschallstrahlen, um eine Bläschenvibrationsamplitude zu erhalten, nahe bei einem spezifizierten Wert.

13. System nach Anspruch 1, mit
- einem Mittel zum Bestimmen einer Relation zwischen der Mikrobläschenvibrationsamplitude und der entsprechenden maximalen Amplitude der Kapillarwandvibrationen.

## Revendications

1. Système (600) permettant d'augmenter une réponse immunitaire contre un cancer existant chez un patient, comprenant
a) des moyens pour une action primaire sur la tumeur primaire du patient qui génère des cellules professionnelles présentatrices d'antigènes APCs/DC avec des antigènes associés à la tumeur (TAA) des cellules cancéreuses primaires dans le fluide interstitiel de la région cancéreuse primaire, et
b) des moyens qui, pendant un délai sélectionnable après le début de ladite action primaire, exécutent une ou plusieurs actions secondaires, lesdits moyens comprenant
i) des moyens d'introduire des microbulles (109) dans le système capillaire tumoral de la région cancéreuse, et
ii) des moyens (608) pour faire vibrer lesdites microbulles avec des faisceaux ultrasonores incidents de fréquence et d'amplitude appropriées, dans le but d'augmenter l'écoulement de liquide lymphatique contenant des APC/DC avec TAA de la tumeur primaire vers les ganglions lymphatiques de drainage (DLN).

2. Système selon la revendication 1, où lesdits moyens d'action primaire comprennent des moyens de thérapie chimique ou radiopharmaceutique.

3. Système selon la revendication 2, où lesdits moyens d'action secondaire comprend des moyens de retarder ladite action secondaire jusqu'à ce que la concentration du médicament chimique ou radio-pharmaceutique dans le sang soit inférieure à une limite définie.

4. Système selon la revendication 1, où lesdits moyens d'action primaire comprennent des moyens de radiothérapie stéréotaxique hypofractionnée ou de protonthérapie.

5. Système selon la revendication 1, où lesdits moyens d'action primaire comprennent des moyens d'action du rayonnement de la curiethérapie.

6. Système selon la revendication 1, où lesdits moyens d'action primaire comprend des moyens d'effectuer de multiples actions primaires à des intervalles de temps sélectionnés,

7. Système selon la revendication 1 ou 6, où lesdits moyens d'action secondaire comprend des moyens d'effectuer ladite action secondaire plusieurs fois après une action primaire.

8. Système selon la revendication 1, où lesdits moyens d'introduire des microbulles comprend des moyens d'injecter des microgouttelettes de fluide dans le sang, et des moyens d'évaporer lesdites microgouttelettes par des ultrasons incidents.

9. Système selon la revendication 1, où lesdits moyens d'introduction de microbulles comprend des moyens d'injection dans le sang de l'une des microbulles suivantes: i) des microbulles d'agent de contraste ultrasonore et ii) des microbulles d'un diamètre supérieur à 5 microns.

10. Système selon la revendication 1, où lesdits moyens de faire vibrer les microbulles comprend
- des moyens de vibration ultrasonique desdites microbulles à une fréquence inférieure à 2 MHz, et
- des moyens d'imagerie de la région cancéreuse et desdites microbulles à une fréquence supérieure à 2 MHz.

11. Système selon la revendication 10, où lesdits moyens d'imagerie comprennent
- des moyens de transmission de complexes d'impulsions comprenant des impulsions basse fréquence (BF) et haute fréquence (HF) copropageant et se chevauchant, lesdites impulsions BF étant utilisées pour manipuler de manière non linéaire les propriétés de l'objet observées par l'impulsion HF copropageant, et
- des moyens d'utiliser la manipulation non linéaire des propriétés de l'objet par l'impulsion BF sur le signal diffusé par l'impulsion HF copropagée pour produire des images de la région cancéreuse et des microbulles.

12. Système selon la revendication 1, où lesdits moyens de faire vibrer les microbulles comprend
- des moyens pour déterminer l'amplitude de vibration desdites microbulles à l'aide d'ultrasons, et
- des moyens d'utiliser l'amplitude mesurée comme donnée d'entrée pour ajuster l'amplitude d'émission desdits faisceaux ultrasonores afin d'obtenir une amplitude de vibration de la bulle proche d'une valeur spécifiée.

13. Système selon la revendication 1, comprenant
- des moyens pour déterminer une relation entre l'amplitude des vibrations des microbulles et l'amplitude maximale correspondante des vibrations de la paroi capillaire.
